# EUROPEAN PATENT SPECIFICATION

(11) **EP 3 030 240 B1**
(45) Date of publication and mention of the grant of the patent: **03.10.2018**
(21) Application number: 14744092.9
(22) Date of filing: 25.07.2014
(51) Int. Cl.: A61K 31/5415, A61K 31/195, A61K 31/17, A61P 27/02

(54) **CARNITINE PALMITOYLTRANSFERASE 1 INHIBITORS FOR INHIBITION OF PATHOLOGICAL ANGIOGENESIS**
CARNITIN-PALMITOYLTRANSFERASE-1-INHIBITOREN ZUR HEMMUNG DER PATHOLOGISCHEN ANGIOGENESE
INHIBITEURS DE CARNITINE PALMITOYLTRANSFÉRASE 1 POUR INHIBER L'ANGIOGENÈSE PATHOLOGIQUE

(30) Priority: 05.08.2013 EP 13179300
(43) Date of publication of application: 15.06.2016
(73) Proprietor: VIB VZW, 9052 Gent (BE); Katholieke Universiteit Leuven K.U. Leuven R&D, 3000 Leuven (BE); Life Sciences Research Partners VZW, 3000 Leuven (BE)
(72) Inventor: CARMELIET, Peter, 3052 Blanden (BE); SCHOORS, Sandra, 2270 Herenthout (BE)
(86) International application number: PCT/EP2014/066024
(87) International publication number: WO 2015/018660

(56) References cited:
- WO-A1-2009/002433
- WO-A1-2010/008472
- US-A1- 2007 191 603
- CARMELIET PETER: "Angiogenesis in health and disease", NATURE MEDICINE, NATURE PUBLISHING GROUP, NEW YORK, NY, US, vol. 9, no. 6, 1 June 2003 (2003-06-01), pages 653-660, XP002422112, ISSN: 1078-8956, DOI: 10.1038/NM0603-653

### SEQUENCE LISTING

<110> VIB VZW
   LIFE SCIENCES RESEARCH PARTNERS VZW
   KATHOLIEKE UNIVERSITEIT LEUVEN, K.U.LEUVEN R&D
<120> Carnitine palmitoyltransferase 1 inhibitors for inhibition of pathological angiogenesis
<130> PCA/CPT/465
<150> EP 13179300.2
   <151> 2013-08-05
<160> 1
<170> PatentIn version 3.5
<210> 1
   <211> 58
   <212> DNA
   <213> Artificial Sequence
<220>
   <223> shRNA
<400> 1
   ccgggccatg aagctcttag acaaactcga gtttgtctaa gagcttcatg gctttttg 58

## Description

### Field of the invention

The present invention relates to the field of pathological angiogenesis such as pathological ocular angiogenesis. In particular the invention has found that inhibitors reducing the activity of the enzyme carnitine palmitoyltransferase 1 can be used for treatment of diseases in which pathological angiogenesis is involved. In particular the invention provides siRNAs directed against carnitine palmitoyltransferase 1 for the treatment of pathological angiogenesis. The invention also provides the use of a therapeutically effective amount of inhibitors of carnitine palmitoyltransferase 1 or a pharmaceutically acceptable salt thereof for the treatment of pathological angiogenesis such as pathological ocular angiogenesis.

### Introduction of the invention

Changes in cellular metabolism and the increased demand for intermediate metabolites and precursors for protein, lipid, and nucleotide synthesis are prerequisites for the invasive, metastatic, and adaptive properties of cancer cells. These metabolic programs may be dictated by specific oncogenic activities. For example, several studies support a direct role for c-myc on mitochondrial functions by indicating that c-myc not only promotes glycolysis but also enhances the ability of mitochondria to use non-glucose substrates, which is essential for the production of cellular metabolic intermediates. Metabolic changes are therefore an optimal target for the development of cancer management therapies. Multiple lines of evidence have documented how fatty acid (FA) synthesis is an important element in cancer cell survival and progression, being sustained by mitochondrial function to produce cytosolic acetyl coenzyme A (acetyl-CoA). The acetyl group of acetyl-CoA is the requisite building block for the endogenous synthesis of FA, cholesterol, and isoprenoids and for the acetylation reactions that modify proteins. Acetyl groups can leave mitochondria in the form of citrate by tricarboxylate transport. In the cytosol, citrate is cleaved by ATP citrate lyase, both to produce cytosolic acetyl-CoA and to regenerate oxaloacetate. To a lesser extent, the oxidative metabolism-derived mitochondrial acetyl-CoA is converted into acetyl-L-carnitine by carnitine acetyltransferase (CAT) before being transported into cytosol by carnitine/acylcarnitine translocase (CACT). Evidence of mitochondrial FA metabolism blockade as a therapeutic approach against cancer already exists and includes the inhibition of carnitine palmitoyltransferase type 1 (CPT1), a mitochondrial enzyme involved in FA channeling inside mitochondria for β-oxidation. Three different isoforms of CPT1 have been identified (CPT1A, CPT1B, and CPT1C), which are differently distributed in organs and tissues.

Endothelial cells (ECs) can survive for several years as quiescent cells in a high-oxygen environment, yet can also rapidly start to proliferate and migrate during vessel sprouting. While the former process requires redox homeostasis, the latter requires the production of energy and biomolecules for DNA, lipid and protein duplication. In general, very little is however known about how ECs adapt their metabolism when initiating vascular branching or resuming to quiescence. Only a few publications reported that ECs are highly glycolytic, while mitochondria are considered to serve primarily signaling purposes. In the present invention we investigated if mitochondrial metabolism in ECs was necessary for vascular branching, and focused on fatty acid oxidation (FAO), since very little is known about the role of this pathway in angiogenesis *in vivo.* We therefore generated EC-specific knockout mice for carnitine palmitoyltransferase 1a (CPT1a), a rate-limiting enzyme of FAO. In these KO mice a severe vascular branching phenotype was observed during postnatal retinal angiogenesis. Furthermore, CPT1a silencing impaired sprouting in an EC spheroid assay, and reduced EC proliferation in vitro. However, energy charge or ATP levels were unaltered and replenishment of the TCA rescued the sprouting defect. The present invention shows that mitochondria in ECs have important metabolic functions necessary for vessel growth and maintenance. More particularly, the present invention can be used to treat pathological angiogenesis by inhibiting the activity of CPT1a.

### Figures

Figure 1: ³H palmitate oxidation assay showing reduced fatty acid oxidation in shCPT1a cells
Figure 2: ³H thymidine incorporation assay showing reduced proliferation in shPT1a cells
Figure 3: Scratch wound assay showing no differences in migration between control and shCPT1a cells.
Figure 4: Representative picture of a WT and CPT1acKO retina showing reduced branching upon knock down of CPT1a in the retinal vessels.
Figure 5: A) EC spheroid sprouting assay showing reduced sprouting upon CPT1a knock down. B) Mitomycin C treated spheroids, to block proliferation, do not show any effect of CPT1a knockdown on sprouting.
Figure 6: Energy charge measurement showing no effect of CPT1a knock down. Furthermore, western blot for AMPK phosphorylation does not show any ATP distress in shCPT1a cells.
Figure 7: Quantification of control and CPT1a knock down spheroids supplemented with 20mM pyruvate or 20mM acetate to replenish the TCA cycle. In control conditions CPT1a knock down reduces sprouting and this effect is rescued upon replenishment of the TCA.
Figure 8: administration of etomoxir reduced the pathological neovascular area when compared to vehicle treated mice (ctrl), (n= 6 mice for ctrl, n = 7 mice for 35 mg/kg; *p<0.05). Panels A and B show a representative image of a control (A) and etomoxir (B) treated CNV lesion, panel C shows the quantification of the CNV area (left is vehicle treated group (ctrl) and right is the etomoxir treated group (eto).

### Detailed description of the invention

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. Unless specifically defined herein, all terms used herein have the same meaning as they would to one skilled in the art of the present invention. Practitioners are particularly directed to Sambrook et al., Molecular Cloning: A Laboratory Manual, 4th ed., Cold Spring Harbor Press, Plainsview, New York (2012); and Ausubel et al., current Protocols in Molecular Biology (Supplement 100), John Wiley & Sons, New York (2012), for definitions and terms of the art. The definitions provided herein should not be construed to have a scope less than understood by a person of ordinary skill in the art.

The present invention shows a role for carnitine palmityoyltransferase 1a (CPT1a) driven fatty acid oxidation in EC proliferation. CPT1a blockade reduced proliferation but not migration *in vitro* and in an EC speroid model the observed sprouting defect was absent when proliferation was blocked using mitomycin C. We showed that this proliferation defect was not due to a decrease in ATP production as no ATP distress was observed. On the other hand, replenishment of the tricarboxylic acid cycle (TCA) using pyruvate or acetate increased sprouting in shRNA downregulated CPT1a spheroids to levels seen in control. Overall these data indicate that CPT1a driven fatty acid oxidation supports the production of TCA intermediates during angiogenesis, necessary for proliferation. The present invention shows that CPT1a is a target for inhibiting pathological angiogenesis.

Carnitine palmitoyltransferase I (CPT1) also known as carnitine acyltransferase I or CAT1 is a mitochondrial enzyme. It is part of a family of enzymes called carnitine acyltransferases. Three isoforms of CPT1 are currently known: CPT1A, CPT1B, and CPT1C. The liver isoform (CPT1A or CPTI-L; UniProtKB/Swis-Prot CPT1A human, P50416) is found throughout the body on the mitochondria of all cells except for skeletal muscle cells and white and brown adipose cells. The muscle isoform (CPT1 B or CPTI-M) is highly expressed in heart and skeletal muscle cells and white and brown adipose cells. A third isoform, the brain isoform (CPT1C) is expressed predominantly in the brain and testes. CPT1a catalyzes the transfer of the acyl group of long-chain fatty acid-CoA conjugates onto carnitine, an essential step for the mitochondrial uptake of long-chain fatty acids and their subsequent beta-oxidation in the mitochondrion. The enzyme plays an important role in triglyceride metabolism.

Possible medical applications of using CPT1a inhibitors have been described in the art for the treatment of cancer, hyperglycemia, obesity, hypertension, insulin resistance syndrome, metabolic syndrome, hyperlipidemia, fatty liver disease, congestive heart failure, renal failure, ischemic disorders, atherosclerosis and psoriasis. The present invention surprisingly shows that the inhibition of carnitine palmitoyltransferase 1A can prevent pathological angiogenesis such as pathological ocular angiogenesis.

Accordingly in a first embodiment the invention provides a compound inhibiting carnitine palmitoyltransferase 1A for treatment of pathological angiogenesis.

In yet another embodiment the invention provides a compound inhibiting carnitine palmitoyltransferase 1A for the treatment of pathological ocular angiogenesis.

The term "pathological ocular angiogenesis" refers to eye (ocular or intraocular) disorders which have an excessive angiogenesis component. A non-limiting list of such diseases is age-related macular degeneration, diabetic retinopathy, diabetic maculopathy and choroidal, proliferative retinopathies.

In the present invention "a compound" inhibiting carnitine palmitoyltransferase 1A encompasses siRNA, ribozymes, shRNA, anti-sense RNA, microRNA directed against carnitine palmitoyltransferase 1A. In addition a "compound" inhibiting carnitine palmitoyltransferase 1A also includes chemical compounds which are able to inhibit the activity of carnitine palmitoyltransferase 1A.

In a particular embodiment a compound is a siRNA with a specificity for carnitine palmitoyltransferase 1A for the treatment of pathological angiogenesis.

In a specific embodiment the siRNA with a specificity for carnitine palmitoyltransferase 1A is expressed by an expression construct incorporated into an adenoviral associated (AAV) vector.

The term "siRNA" refers to a small interfering RNA(s), which also has been referred to in the art as short interfering RNA and silencing RNA, among others. siRNAs generally are described as relatively short, often 20-25 nucleotide-long, double-stranded RNA molecules that are involved in RNA interference (RNAi) pathway(s). Generally, siRNAs are, in part, complementary to specific mRNAs (such as carnitine palmitoyltransferase 1A) and mediate their down regulation (hence, "interfering"). siRNAs thus can be used for down regulating the expression of specific genes and gene function in cells and organisms. siRNAs also play a role in related pathways. The general structure of most naturally occurring siRNAs is well established. Generally, siRNAs are short double-stranded RNAs, usually 21 nucleotides long, with two nucleotides single stranded "overhangs" on the 3 of each strand. Each strand has a 5' phosphate group and a 3' hydroxyl (--OH) group. In vivo, the structure results from processing by the enzyme "dicer," which enzymatically converts relatively long dsRNAs and relatively small hairpin RNAs into siRNAs. The term siNA refers to a nucleic acid that acts like a siRNA, as described herein, but may be other than an RNA, such as a DNA, a hybrid RNA:DNA or the like. siNAs function like siRNAs to down regulate expression of gene products. The term "RNA interference" which also has been called "RNA mediated interference" refers to the cellular processes by which RNA (such as siRNAs) down regulate expression of genes; i.e., down regulate or extinguish the expression of gene functions, such as the synthesis of a protein encoded by a gene. Typically, double-stranded ribonucleic acid inhibits the expression of genes with complementary nucleotide sequences. RNA interference pathways are conserved in most eukaryotic organisms. It is initiated by the enzyme dicer, which cleaves RNA, particularly double-stranded RNA, into short double-stranded fragments 20-25 base pairs long. One strand of the double-stranded RNA (called the "guide strand") is part of a complex of proteins called the RNA-induced silencing complex (RISC). The thus incorporated guide strand serves as a recognition sequence for binding of the RISC to nucleic acids with complementary sequences. Binding by RISC to complementary nucleic acids results in their being "silenced." The best studied silencing is the binding of RISCs to RNAs resulting in post-transcriptional gene silencing. Regardless of mechanism, interfering nucleic acids and RNA interference result in down regulation of the target gene or genes that are complementary (in pertinent part) to the guide strand. A polynucleotide can be delivered to a cell to express an exogenous nucleotide sequence, to inhibit, eliminate, augment, or alter expression of an endogenous nucleotide sequence, or to affect a specific physiological characteristic not naturally associated with the cell. The polynucleotide can be a sequence whose presence or expression in a cell alters the expression or function of cellular genes or RNA.

In addition, the present invention contemplates polynucleotide-based expression inhibitors of carnitine palmitoyltransferase 1A which may be selected from the group comprising: siRNA, microRNA, interfering RNA or RNAi, dsRNA, ribozymes, antisense polynucleotides, and DNA expression cassettes encoding siRNA, microRNA, dsRNA, ribozymes or antisense nucleic acids. SiRNA comprises a double stranded structure typically containing 15 to 50 base pairs and preferably 19 to 25 base pairs and having a nucleotide sequence identical or nearly identical to an expressed target gene or RNA within the cell. An siRNA may be composed of two annealed polynucleotides or a single polynucleotide that forms a hairpin structure. MicroRNAs (miRNAs) are small noncoding polynucleotides, about 22 nucleotides long, that direct destruction or translational repression of their mRNA targets. Antisense polynucleotides comprise a sequence that is complimentary to a gene or mRNA. Antisense polynucleotides include, but are not limited to: morpholinos, 2'-O-methyl polynucleotides, DNA, RNA and the like. The polynucleotide-based expression inhibitor may be polymerized in vitro, recombinant, contain chimeric sequences, or derivatives of these groups. The polynucleotide-based expression inhibitor may contain ribonucleotides, deoxyribonucleotides, synthetic nucleotides, or any suitable combination such that the target RNA and/or gene is inhibited. Polynucleotides may contain an expression cassette coded to express a whole or partial protein, or RNA. An expression cassette refers to a natural or recombinantly produced polynucleotide that is capable of expressing a sequence. The cassette contains the coding region of the gene of interest along with any other sequences that affect expression of the sequence of interest. An expression cassette typically includes a promoter (allowing transcription initiation), and a transcribed sequence. Optionally, the expression cassette may include, but is not limited to, transcriptional enhancers, non-coding sequences, splicing signals, transcription termination signals, and polyadenylation signals. An RNA expression cassette typically includes a translation initiation codon (allowing translation initiation), and a sequence encoding one or more proteins. Optionally, the expression cassette may include, but is not limited to, translation termination signals, a polyadenosine sequence, internal ribosome entry sites (IRES), and non-coding sequences. The polynucleotide may contain sequences that do not serve a specific function in the target cell but are used in the generation of the polynucleotide. Such sequences include, but are not limited to, sequences required for replication or selection of the polynucleotide in a host organism.

Based on the RNA sequence of carnitine palmitoyltransferase 1A, siRNA molecules with the ability to knock-down carnitine palmitoyltransferase 1A activity can be obtained by chemical synthesis or by hairpin siRNA expression vectors. There are numerous companies that provide the supply of costumer-designed siRNAs on a given RNA sequence, e.g. Ambion, Imgenex, Dharmacon.

The carnitine palmitoyltransferase 1A siRNAs of the invention may be chemically modified, e.g. as described or example in US20030143732, by phosphorothioate internucleotide linkages, 2'-O-methyl ribonucleotides, 2'-deoxy-2'fluoro ribonucleotides, "universal base" nucleotides, 5-C-methyl nucleotides, and inverted deoxyabasic residue incorporation. The sense strand carnitine palmitoyltransferase 1A siRNAs may also be conjugated to small molecules or peptides, such as membrane-permeant peptides or polyethylene glycol (PEG). Other siRNA conjugates which form part of the present invention include cholesterol and alternative lipid-like molecules, such as fatty acids or bile-salt derivatives.

In a further embodiment, the present invention relates to an expression vector comprising any of the above described polynucleotide sequences encoding at least one carnitine palmitoyltransferase 1A siRNA molecule in a manner that allows expression of the nucleic acid molecule, and cells containing such vector. The polynucleic acid sequence is operably linked to regulatory signals (promoters, enhancers, suppressors etc.) enabling expression of the polynucleic acid sequence and is introduced into a cell utilizing, preferably, recombinant vector constructs. A variety of viral-based systems are available, including adenoviral, retroviral, adeno-associated viral, lentiviral, herpes simplex viral vector systems. Selection of the appropriate viral vector system, regulatory regions and host cell is common knowledge within the level of ordinary skill in the art.

As gene delivery and gene silencing techniques improve, the selective deletion of carnitine palmitoyltransferase 1A, for example in the eye, may prove useful in order to limit the impact of protein deletion to a particular system under study. The carnitine palmitoyltransferase 1A siRNA molecules of the invention may be delivered by known gene delivery methods, e.g. as described in US20030143732, including the use of naked siRNA, synthetic nanoparticles composed of cationic lipid formulations, liposome formulations including pH sensitive liposomes and immunoliposomes, or bioconjugates including siRNAs conjugated to fusogenic peptides. Delivery of siRNA expressing vectors can also be systemic, such as by intravenous, intraperitoneal, intraocular, intravitreal or intramuscular administration or even by intrathecal or by intracerebral injection that allows for introduction into the desired target cell (see US 20030143732).

In yet another embodiment the compound inhibiting carnitine palmitoyltransferase 1A is a chemical compound able to inhibit the enzyme carnitine palmitoyltransferase 1A for the treatment of a pathological angiogenesis, excluding cancer. In specific embodiments the previous compounds (e.g. siRNAs and chemical compounds) for the treatment of pathological angiogenesis - excluding cancer - are used for the treatment of age-related macular degeneration, diabetic retinopathy, diabetic maculopathy, choroidal, proliferative retinopathies and other intraocular disorders with an excessive angiogenesis component. The term "excessive angiogenesis component with respect to intraocular disorders" has the same meaning as "pathological ocular angiogenesis" and refers to the fact that in certain pathological eye diseases, such as described herein before, an excess angiogenesis occurs. A medical doctor such as an eye doctor or eye surgeon is well positioned to determine if excessive pathological ocular angiogenesis occurs in the eye.

In yet another embodiment the invention provides a siRNA with a specificity for carnitine palmitoyltransferase 1A for the treatment of conditions and disorders resulting from pathological angiogenesis including diseases from the list macular degeneration, and proliferative retinopathies.

In a specific embodiment said siRNA with a specificity for carnitine palmitoyltransferase 1A is expressed by an expression construct incorporated into a viral vector.

In yet another specific embodiment said siRNA with a specificity for carnitine palmitoyltransferase 1A is expressed by an expression construct incorporated into an adenoviral-2 associated (AAV-2) vector.

The instant invention provides a method of reducing angiogenesis in a mammal. The method generally involves administering to a mammal a siRNA with a specificity for carnitine palmitoyltransferase 1A and/or a compound as herein described before which inhibits the enzyme carnitine palmitoyltransferase 1A in an amount effective to reduce angiogenesis. An effective amount of a siRNA with a specificity for carnitine palmitoyltransferase 1A, in combination with, or applied separately with a compound as herein described before which inhibits the enzyme carnitine palmitoyltransferase 1A, reduces angiogenesis by at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, or more, when compared to an untreated (e.g. a placebo-treated) control. Whether angiogenesis is reduced can be determined using any known method. Methods of determining an effect of an agent on angiogenesis are known in the art and include, but are not limited to, inhibition of neovascularization into implants impregnated with an angiogenic factor; inhibition of blood vessel growth in the cornea or anterior eye chamber; inhibition of endothelial cell proliferation, migration or tube formation in vitro; the chick chorioallantoic membrane assay; the hamster cheek pouch assay; the polyvinyl alcohol sponge disk assay; the formation of blood vessels in zebrafish larvae. Such assays are well known in the art and have been described in numerous publications.

The term "pathological angiogenesis" as used herein refers to the excessive formation and growth of blood vessels during the maintenance and the progression of several disease states. Examples where pathological angiogenesis can occur are blood of the eye (retinopathy of prematurity, diabetic retinopathy, choroidal and other intraocular disorders (e.g. macular degeneration), leukomalacia), heart and skeletal muscle due to work overload, adipose tissue (obesity), endocrine organs (thyroiditis, thyroid enlargement, pancreas transplantation). While it is generally known in the art that pathological angiogenesis is also associated with neoplasms and metastasis, the latter conditions are herein specifically excluded (or disclaimed) from the claim scope of the invention.

Chemical compounds inhibiting the activity of carnitine palmitoyltransferase 1A are well known in the art and comprise sulfonamides as described and claimed in US20120232104, heterobicyclic sulfonamide derivatives as described and claimed in EP1996563B1, sulfonamides as described and claimed in US20110319438, substituted aminocarnitine compounds as described and claimed in US20110230555, piperidine-amide compounds as described and claimed in EP2155738B1, inhibitors as described and claimed in US20100210695, sulfonamide compounds as described and claimed in EP2097373B1, sulfonamide derivatives as described and claimed in EP1891001B1, sulfonamides as described and claimed in US20100144762, heteroaryl substituted piperidine derivatives as described and claimed in EP1959951B1, heterobicyclic derivatives as described and claimed in EP1926711, indolyl derivatives as described and claimed in US20070060567, inhibitors described and claimed in WO1997000678. It is understood that these referenced CPT1a chemical inhibitors are useful for treatment of pathological angiogenesis excluding cancer and psoriasis; more particularly are useful for treating pathological ocular angiogenesis.

### Medicinal uses:

This invention also relates to pharmaceutical compositions containing one or more compounds of the present invention. These compositions can be utilized to achieve the desired pharmacological effect by administration to a patient in need thereof. A patient, for the purpose of this invention, is a mammal, including a human, in need of treatment for the particular condition or disease, i.e. a disease wherein pathological angiogenesis is involved excluding cancer (excluding tumors or excluding neoplasia which are equivalent terms). Therefore, the present invention includes pharmaceutical compositions that are comprised of a pharmaceutically acceptable carrier and a pharmaceutically effective amount of a compound, or salt thereof, of the present invention. A pharmaceutically acceptable carrier is preferably a carrier that is relatively non-toxic and innocuous to a patient at concentrations consistent with effective activity of the active ingredient so that any side effects ascribable to the carrier do not vitiate the beneficial effects of the active ingredient. A pharmaceutically effective amount of compound is preferably that amount which produces a result or exerts an influence on the particular condition being treated. The compounds of the present invention can be administered with pharmaceutically-acceptable carriers well known in the art using any effective conventional dosage unit forms, including immediate, slow and timed release preparations, orally, intraperitoneally, parenterally, topically, nasally, ophthalmically, optically, sublingually, rectally, vaginally, intrathecally, intracerebroventricularly and the like.

For oral administration, the compounds can be formulated into solid or liquid preparations such as capsules, pills, tablets, troches, lozenges, melts, powders, solutions, suspensions, or emulsions, and may be prepared according to methods known to the art for the manufacture of pharmaceutical compositions. The solid unit dosage forms can be a capsule that can be of the ordinary hard- or soft-shelled gelatin type containing, for example, surfactants, lubricants, and inert fillers such as lactose, sucrose, calcium phosphate, and corn starch.

In another embodiment, the compounds of this invention may be tableted with conventional tablet bases such as lactose, sucrose and cornstarch in combination with binders such as acacia, corn starch or gelatin, disintegrating agents intended to assist the break-up and dissolution of the tablet following administration such as potato starch, alginic acid, corn starch, and guar gum, gum tragacanth, acacia, lubricants intended to improve the flow of tablet granulation and to prevent the adhesion of tablet material to the surfaces of the tablet dies and punches, for example talc, stearic acid, or magnesium, calcium or zinc stearate, dyes, coloring agents, and flavoring agents such as peppermint, oil of wintergreen, or cherry flavoring, intended to enhance the aesthetic qualities of the tablets and make them more acceptable to the patient. Suitable excipients for use in oral liquid dosage forms include dicalcium phosphate and diluents such as water and alcohols, for example, ethanol, benzyl alcohol, and polyethylene alcohols, either with or without the addition of a pharmaceutically acceptable surfactant, suspending agent or emulsifying agent. Various other materials may be present as coatings or to otherwise modify the physical form of the dosage unit. For instance tablets, pills or capsules may be coated with shellac, sugar or both.

Dispersible powders and granules are suitable for the preparation of an aqueous suspension. They provide the active ingredient in admixture with a dispersing or wetting agent, a suspending agent and one or more preservatives. Suitable dispersing or wetting agents and suspending agents are exemplified by those already mentioned above. Additional excipients, for example those sweetening, flavoring and coloring agents described above, may also be present.

The pharmaceutical compositions of this invention may also be in the form of oil-in-water emulsions. The oily phase may be a vegetable oil such as liquid paraffin or a mixture of vegetable oils. Suitable emulsifying agents may be (1) naturally occurring gums such as gum acacia and gum tragacanth, (2) naturally occurring phosphatides such as soy bean and lecithin, (3) esters or partial esters derived from fatty acids and hexitol anhydrides, for example, sorbitan monooleate, (4) condensation products of said partial esters with ethylene oxide, for example, polyoxyethylene sorbitan monooleate. The emulsions may also contain sweetening and flavoring agents.

Oily suspensions may be formulated by suspending the active ingredient in a vegetable oil such as, for example, arachis oil, olive oil, sesame oil or coconut oil, or in a mineral oil such as liquid paraffin. The oily suspensions may contain a thickening agent such as, for example, beeswax, hard paraffin, or cetyl alcohol. The suspensions may also contain one or more preservatives, for example, ethyl or n-propyl p-hydroxybenzoate; one or more coloring agents; one or more flavoring agents; and one or more sweetening agents such as sucrose or saccharin. Syrups and elixirs may be formulated with sweetening agents such as, for example, glycerol, propylene glycol, sorbitol or sucrose. Such formulations may also contain a demulcent, and preservative, such as methyl and propyl parabens and flavoring and coloring agents.

The parenteral compositions of this invention will typically contain from about 0.5% to about 25% by weight of the active ingredient in solution. Preservatives and buffers may also be used advantageously. In order to minimize or eliminate irritation at the site of injection, such compositions may contain a non-ionic surfactant having a hydrophile-lipophile balance (HLB) preferably of from about 12 to about 17. The quantity of surfactant in such formulation preferably ranges from about 5% to about 15% by weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The pharmaceutical compositions may be in the form of sterile injectable aqueous suspensions. Such suspensions may be formulated according to known methods using suitable dispersing or wetting agents and suspending agents such as, for example, sodium carboxymethylcellulose, methylcellulose, hydroxypropylmethyl-cellulose, sodium alginate, polyvinylpyrrolidone, gum tragacanth and gum acacia ; dispersing or wetting agents which may be a naturally occurring phosphatide such as lecithin, a condensation product of an alkylene oxide with a fatty acid, for example, polyoxyethylene stearate, a condensation product of ethylene oxide with a long chain aliphatic alcohol, for example, heptadeca-ethyleneoxycetanol, a condensation product of ethylene oxide with a partial ester derived form a fatty acid and a hexitol such as polyoxyethylene sorbitol monooleate, or a condensation product of an ethylene oxide with a partial ester derived from a fatty acid and a hexitol anhydride, for example polyoxyethylene sorbitan monooleate.

The sterile injectable preparation may also be a sterile injectable solution or suspension in a non-toxic parenterally acceptable diluent or solvent. Diluents and solvents that may be employed are, for example, water, Ringer's solution, isotonic sodium chloride solutions and isotonic glucose solutions. In addition, sterile fixed oils are conventionally employed as solvents or suspending media. For this purpose, any bland, fixed oil may be employed including synthetic mono- or diglycerides. In addition, fatty acids such as oleic acid can be used in the preparation of injectables.

In a particular embodiment the pharmaceutical composition of the invention is an ocular (or ophthalmic) pharmaceutical composition.

A composition of the invention may also be administered in the form of suppositories for rectal administration of the drug. These compositions can be prepared by mixing the drug with a suitable non-irritation excipient which is solid at ordinary temperatures but liquid at the rectal temperature and will therefore melt in the rectum to release the drug. Such materials are, for example, cocoa butter and polyethylene glycol.

Another formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art (see for example US 5,023,252). Such patches may be constructed for continuous, pulsatile, or on demand delivery of pharmaceutical agents. Controlled release formulations for parenteral administration include liposomal, polymeric microsphere and polymeric gel formulations that are known in the art. It may be desirable or necessary to introduce the pharmaceutical composition to the patient via a mechanical delivery device. The construction and use of mechanical delivery devices for the delivery of pharmaceutical agents is well known in the art. Direct techniques for, for example, administering a drug directly to the brain usually involve placement of a drug delivery catheter into the patient's ventricular system to bypass the blood-brain barrier. One such implantable delivery system, used for the transport of agents to specific anatomical regions of the body, is described in US 5,011,472.

The compositions of the invention can also contain other conventional pharmaceutically acceptable compounding ingredients, generally referred to as carriers or diluents, as necessary or desired. Conventional procedures for preparing such compositions in appropriate dosage forms can be utilized. Such ingredients and procedures include those described in the following references, each of which is incorporated herein by reference: Powell, M. F. et al., "Compendium of Excipients for Parenteral Formulations" PDA Journal of Pharmaceutical Science & Technology 1998, 52(5), 238-311 ; Strickley, R.G "Parenteral Formulations of Small Molecule Therapeutics Marketed in the United States (1999)-Part-1" PDA Journal of Pharmaceutical Science & Technology 1999, 53(6), 324-349 ; and Nema, S. et al. , "Excipients and Their Use in Injectable Products" PDA Journal of Pharmaceutical Science & Technology 1997, 51 (4), 166-171.

In a specific embodiment ocular delivery (or delivery to the eye) is preferred. For local delivery to the eye, the pharmaceutically acceptable compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutically acceptable compositions may be formulated in an ointment such as petrolatum. Preferred methods of local ocular administration include e.g. choroidal injection, transscleral injection or placing a scleral patch, selective arterial catheterization, intraocular administration including transretinal, subconjunctival bulbar, intravitreous injection, suprachoroidal injection, subtenon injection, scleral pocket and scleral cutdown injection, by osmotic pump, etc. In choroidal injection and scleral patching, the clinician uses a local approach to the eye after initiation of appropriate anesthesia, including painkillers and ophthalmoplegics. A needle containing the therapeutic composition of the invention is directed into the subject's choroid or sclera and inserted under sterile conditions. When the needle is properly positioned the compound is injected into either or both of the choroid or sclera. When using either of these methods, the clinician can choose a sustained release or longer acting formulation. Thus, the procedure can be repeated only every several months, depending on the subject's tolerance of the treatment and response. Intraocular administration of drugs intended for treatment of macular degeneration and other intraocular conditions is well known in the art. See, e.g. U.S. Pat. Nos. 5,632,984 and 5,770,589. U.S. Pat. No. 6,378,526 provides methods for intrascleral injection of a therapeutic at a location overlying the retina, which provide a minimally invasive technique for delivering the agent to the posterior segment of the eye. In certain embodiments of the invention a composition is delivered to the vicinity of the eye, e.g. in close proximity to the posterior segment of the eye. The "vicinity of the eye" refers to locations within the orbit, which is the cavity within the skull in which the eye and its appendages are situated. Typically the compositions would be delivered close to their intended target within the eye, e.g. close to (within several millimeters of) the portion of the sclera that overlies the posterior segment of the eye, or immediately adjacent to the exterior surface of the sclera. A number of polymeric delivery vehicles for providing controlled release have been used in an ocular context and can be used to administer the compositions of the invention. Various polymers, e.g., biocompatible polymers, which may be biodegradable, can be used. For example, U.S. Pat. No. 6,692,759 describes methods for making an implantable device for providing controlled release of therapeutic agents in the eye. Other useful polymers and delivery systems for ocular administration of a therapeutic agent have been described. The active agent may be released as the polymer degrades. Polymers that have been used for drug delivery include, but are not limited to, poly(lactic-co-glycolic acid), polyanhydrides, ethylene vinyl acetate, polyglycolic acid, chitosan, polyorthoesters, polyethers, polylactic acid, and poly (beta amino esters). Peptides, proteins such as collagen and albumin, and dendrimers (e.g., PAMAM dendrimers) have also been used. Any of these can be used in various embodiments of the invention. Poly(ortho-esters) have been introduced into the eye and demonstrated favorable properties for sustained release ocular drug delivery (Einmahl S. (2002), Invest. Ophthalmol. Vis. Sci., 43(5). Polylactide particles have been used to target an agent to the retina and RPE following intravitreous injection of a suspension of such particles (Bourges, J.L. et al (2003) Invest. Ophthalmol. Vis. Sci., 44(8). A macroscopic implantable device suitable for introduction into the posterior or anterior segment of the eye is referred to herein as an ocular implant (Jaffe, G. (2000) Invest. Ophthalmol. Vis. Sci., 41(11). Such devices may be comprised of a plurality of nanoparticles less than or microparticles impregnated with the agent. Methods for making microparticles and nanoparticles are known in the art. Generally, a microparticle will have a diameter of 500 microns or less, e.g., between 50 and 500 microns, between 20 and 50 microns, between 1 and 20 microns, between 1 and 10 microns, and a nanoparticle will have a diameter of less than 1 micron. Preferably the device is implanted into the space occupied by the vitreous humor. The ocular implant may comprise a polymeric matrix. The invention also provides periocular implants, which are macroscopic implantable device suitable for introduction in the vicinity of the eye, e.g., in close proximity to the eye. In certain embodiments the periocular implant is made of similar materials to those described above.

Pharmaceutical compositions according to the present invention can be illustrated as follows:
- Sterile IV Solution: A 5 mg/mL solution of the desired compound of this invention can be made using sterile, injectable water, and the pH is adjusted if necessary. The solution is diluted for administration to 1 - 2 mg/mL with sterile 5% dextrose and is administered as an IV infusion over about 60 minutes.
- Lyophilised powder for IV administration: A sterile preparation can be prepared with (i) 100 - 1000 mg of the desired compound of this invention as a lyophilised powder, (ii) 32- 327 mg/mL sodium citrate, and (iii) 300 - 3000 mg Dextran 40. The formulation is reconstituted with sterile, injectable saline or dextrose 5% to a concentration of 10 to 20 mg/mL, which is further diluted with saline or dextrose 5% to 0.2 - 0.4 mg/mL, and is administered either IV bolus or by IV infusion over 15 - 60 minutes.

Intramuscular suspension: The following solution or suspension can be prepared, for intramuscular injection:
50 mg/mL of the desired, water-insoluble compound of this invention
5 mg/mL sodium carboxymethylcellulose
4 mg/mL TWEEN 80
9 mg/mL sodium chloride
9 mg/mL benzyl alcohol

### Combination therapies

The compounds of this invention can be administered as the sole pharmaceutical agent or in combination with one or more other pharmaceutical agents where the combination causes no unacceptable adverse effects. The present invention relates also to such combinations. For example, the compounds of this invention can be combined with other anti-angiogenic agents. Anti-angiogenic agents include, but are not limited to, angiostatic steroids such as heparin derivatives and glucocorticosteroids; thrombospondin; cytokines such as IL-12; fumagillin and synthetic derivatives thereof, such as AGM 12470; interferon-alpha; endostatin; soluble growth factor receptors; neutralizing monoclonal antibodies directed against growth factors such as vascular endothelial growth factor and the like.

### Dose and administration

Based upon standard laboratory techniques known to evaluate compounds useful for the treatment of diseases where excessive (or pathological) angiogenesis occurs, by standard toxicity tests and by standard pharmacological assays for the determination of treatment of the conditions identified above in mammals, and by comparison of these results with the results of known medicaments that are used to treat these above described conditions, the effective dosage of the compounds of this invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The total amount of the active ingredient to be administered will generally range from about 0.001 mg/kg to about 200 mg/kg body weight per day, and preferably from about 0.01 mg/kg to about 20 mg/kg body weight per day. Clinically useful dosing schedules will range from one to three times a day dosing to once every four weeks dosing. In addition, "drug holidays" in which a patient is not dosed with a drug for a certain period of time, may be beneficial to the overall balance between pharmacological effect and tolerability. A unit dosage may contain from about 0.5 mg to about 150 mg of active ingredient, and can be administered one or more times per day or less than once a day. The average daily dosage for administration by injection, including intravenous, intramuscular, intraocular, intravitreal, subcutaneous, intrathecal, intraceroventricularly and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight. The average daily topical dosage regimen will preferably be from 0.1 to 200 mg administered between one to four times daily. The transdermal concentration will preferably be that required to maintain a daily dose of from 0.01 to 200 mg/kg. The average daily inhalation dosage regimen will preferably be from 0.01 to 100 mg/kg of total body weight.

It is evident for the skilled artesan that the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

It is to be understood that although particular embodiments, specific configurations as well as materials and/or molecules, have been discussed herein for cells and methods according to the present invention, various changes or modifications in form and detail may be made without departing from the scope and spirit of this invention. The following examples are provided to better illustrate particular embodiments, and they should not be considered limiting the application. The application is limited only by the claims.

### Examples

### 1.Carnitine palmitoyl transferase 1A (CPT1a) is of crucial importance for fatty acid oxidation (FAO) in endothelial cells (ECs)

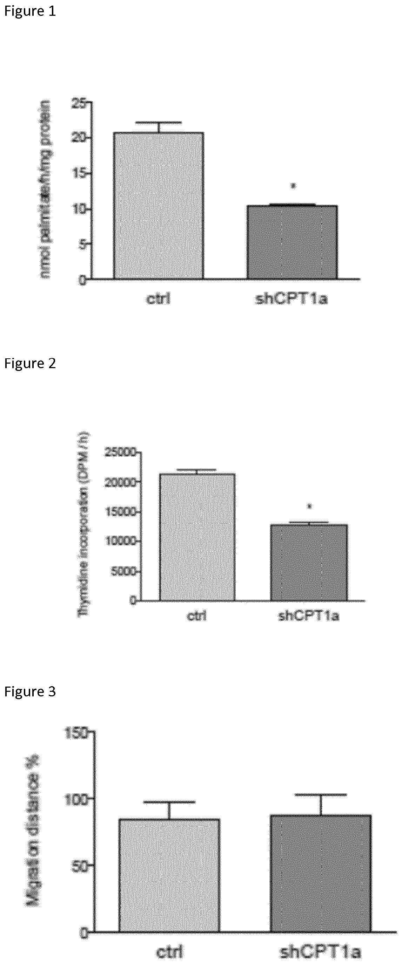

CPT1 enzymes are rate limiting for fatty acid oxidation and of the 3 isoforms, CPT1a is the most abundant in ECs. To explore the functional relevance of CPT1a in ECs, we compared its expression in quiescent versus angiogenic EC monolayers. Strikingly, CPT1a RNA and protein levels were increased during contact inhibition - an *in vitro* model of quiescence - when compared to proliferating/migrating ECs. Similar results were obtained when using notch signaling as an *in vitro* model of quiescence. To correlate these expression levels to functional relevance, fatty acid oxidation was measured using a ³H-9,10-palmitic acid tracer. In accordance with the expression data, quiescent ECs had a significantly higher fatty acid oxidation compared to angiogenic ECs. To confirm that fatty acid oxidation in ECs is primarily CPT1a - driven, we blocked its expression using a shRNA directed against CPT1a. The shRNA sequence we used was 5'-CCGGGCCATGAAGCTCTTAGACAAACTCGAGTTTGTCTAAGAGCTTCATGGCTTTTTG-3'. This specific downregulation of the CPT1a mRNA reduced CPT1a protein levels to nearly undetectable levels and also the fatty acid oxidation was significantly reduced (see Figure 1). These results were also confirmed using the pharmacological blocker etomoxir, which blocks both CPT1a and b. Even more, using notch signaling as a model of quiescence, the notch induced increase in FAO was abrogated upon CPT1a KD. Altogether, these data show that quiescent ECs rely more on FAO compared to proliferating ECs and that ECs in general primarily rely on CPT1a driven fatty acid oxidation.

### 2. CPT1a-driven fatty acid oxidation affects in vitro vessel sprouting in a proliferation dependent manner.

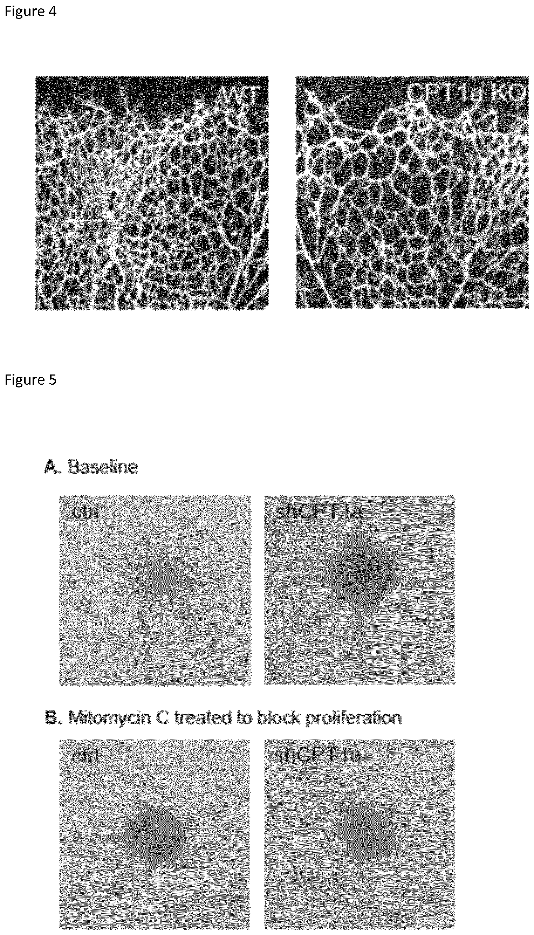

In angiogenic EC monolayers, CPT1a knock down (KD) reduced EC proliferation by 40% as measured by ³H thymidine incorporation (see Figure 2). Furthermore, CPT1a KD EC were found more frequently in the G0/G1 phase of the cell cycle. EC migration on the other hand was not affected as measured by scratch wound assay and modified boyden chamber even when proliferation was blocked using mitomycin C (see Figure 3). To study the effect of CPT1a KD on vessel sprouting we employed an *in vitro* EC spheroid sprouting assay. In this model, EC are cultured in suspension to form spheroids in hanging drops, embedded in a collagen I matrix and subsequently stimulated with growth factors to allow sprouts to be formed. In this model, a tip cell with filopodia leads the sprout while the stalk cells trail behind and proliferate to elongate the sprout. Upon CPT1a knockdown (KD), EC spheroids formed fewer and shorter sprouts however this effect was abrogated upon mitoC treatment (see Figure 5). Similar results were obtained using the CPT1 blocker etomoxir. These data suggest that the effect of CPT1a KD on sprouting is proliferation dependent, in accordance with the absence of a migration defect *in vitro.* To further investigate this observation, we employed a mosaic spheroid model to assess tip cell competition. Here 50% WT^{RED} and 50% WT^{GFP} cells are mixed and when the spheroids have sprouted, we count which one is at the tip. In this model, tip cell position can be determined via migration and/or proliferation. In case of a WT/WT mixture, 50% green and 50% red cells are at the tip. However when 50% WT^{RED} cells are mixed with 50% CPT1a^{KD/GFP} cells, less green cells were present at the tip and this effect was abrogated when spheroids were treated with mitoC.

### 3. CPT1a-driven fatty acid oxidation affects vessel sprouting in vivo

To assess the effect of CPT1a driven FAO on vessel formation *in vivo,* we generated CPT1a^{lox/lox} mice and crossed them with VE-cadherin (PAC)-Cre^{ERT2} mice, an EC specific Cre driver line. In these CPT1a^{ΔEC} mice, we assessed postnatal retinal angiogenesis. Pups were injected with tamoxifen from postnatal day P1-P4 and dissected at P5. EC loss of CPT1a in these pups did not affect body weight or radial expansion of the retinal vasculature but did reduce the amount of branch points, filopodia and distal sprouts with filopodia (see Figure 4). In addition, proliferation in the retinal vasculature was decreased as assessed by Edu incorporation while the amount of empty collagen IV sleeves was increased indicating vessel regression. Pericyte coverage, as measured by NG2 staining was reduced in the CPT1a cKO mice, indicating reduced quiescence in these vessels. Similar results were obtained using etomoxir, which significantly reduced ¹⁴C palmitate uptake in organs the significant incorporation of ¹³C palmitate in TCA intermediates in organs. The body weight of etomoxir treated pups was similar to that of controls and the treatment did not cause any defects in the heart as shown by H&E staining.

Next we generated CPT1a^{lox/lox} - VECadherin-Cre^{ERT2} - mCherry⁺ ES cells and CPT1a^{lox/lox} - mCherry⁺ ES cells which were injected into WT blastocysts. After implantation into pseudo pregnant females, we treated the mice with tamoxifen for 5 days before the due date. Pups were dissected at postnatal day P1 and P5, and the contribution of the Cherry⁺ transgenic cells to the retinal vasculature was assessed. An equal amount of WT^{RED} and CPT1a^{KO/RED} cells were found in P1 retinae, however at P5 only few CPT1a^{KO/RED} cells were detected in the vessels while WT^{RED} cells were still abundantly present. Furthermore, at P1, an equal number of CPT1a^{KO/RED} EC were present at the tip position as WT^{RED} EC but at P5 the CPT1a^{KO/RED} were progressively outcompeted. This was due to a proliferation defect upon CPT1a knock down as significantly less CPT1a^{KO/RED} EC were Edu+ at P5 when compared to WT^{RED} EC.

### 4. CPT1a blockade does not induce ATP distress

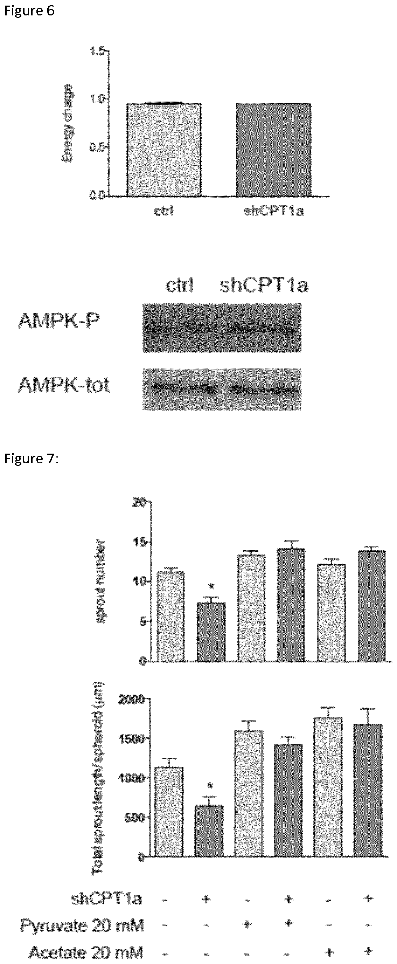

We next questioned how fatty acid oxidation could exert its proliferation dependent effects on vessel sprouting. Through fueling the TCA with 129 molecules of acetyl-COA, 1 palmitate can generate much more ATP via oxidative phosphorylation compared to 1 glucose. However as we have shown previously, angiogenic ECs rely primarily on glycolysis for ATP production. Nevertheless we wanted to exclude that the CPT1a induced proliferation defect was caused by ATP depletion. CPT1a KD did not affect the energy charge nor did it affect total ATP levels. Using the GO-ATeam biosensor for live ATP imaging, we also showed that CPT1a blockade did not induce any drop in ATP signal in the cytosol or the lamellipodia, contrary to what we have shown previously for PFKFB3 KD. One would also expect that whenever cells are in ATP distress, they would upregulate a major ATP producing pathway. However CPT1a blockade did not affect glycolysis in ECs. Furthermore, we did not observe any signs of energy distress, as shown by AMPK-p and no induction of autophagy as measured by LC3. We therefore conclude that blocking CPT1a driven FAO does not affect proliferation due to a reduction in ATP (see Figure 6).

### 5. CPT1a blockade reduces TCA intermediates thereby blocking proliferation

Proliferation requires many building blocks such as protein, RNA, DNA, ... to allow duplication of 1 cell into 2 daughter cells. Up until now, the role of mitochondria in ECs was thought to be for signaling purposes and not so much for biomass production as ECs are highly glycolytic. Nevertheless blocking CPT1a driven FAO reduced proliferation by 40%. FAO can fuel the TCA cycle with 129 molecules of acetyl COA thereby providing plenty of intermediates for biomass as well as producing NADPH that can be used for anti-oxidant defense or lipid synthesis. In addition to a 40% decrease in proliferation, CPT1a blockade also induced a 40% increase in intracellular H₂O₂ levels. However this increase in ROS could not explain the proliferation defect as no DNA damage checkpoint was activated in these cells as measured by ATM-P, p53 and p21 levels. In addition, lowering ROS levels using the anti-oxidant NAC, to levels observed in control did not restore proliferation upon CPT1a KD in EC monolayers or spheroids. We therefore hypothesized that blocking FAO would reduce TCA intermediates needed for biomass production. Even more, using ¹³C palmitate we showed that CPT1a KD cells incorporate less 13C label in TCA intermediates and in biomass. To assess whether the proliferation defect could be rescued by refueling the TCA cycle, we supplemented the cells with pyruvate or acetate, both known to increase oxygen consumption and proliferation. Indeed supplementation of either of these metabolites rescued the proliferation defect in EC monolayers as well as in spheroids, showing that CPT1A driven fatty acid oxidation is of critical importance for the generation of biomass necessary for proliferation (see Figure 7).

### 6. CPT1a differentially affects vessel sprouting and quiescence

One of our striking initial observations was that quiescent ECs have a much higher FAO flux compared to proliferating ECs. Our data now show that FAO generates TCA intermediates to fuel biomass production and thus supports proliferation during vessel sprouting. However this would be an unlikely function of FAO during EC quiescence, where no proliferation is needed. Another major role of FAO is the production of NADPH via malic enzyme or isocitrate dehydrogenase. Indeed we found that quiescent ECs had lower intracellular ROS levels compared to proliferating ECs, in accordance their NADPH levels, as measured by HPLC/MS. Next we induced quiescence in EC spheroids using NICD^{OE} and assessed the effect of altered FAO. Strikingly, silencing of CPT1a further reduced sprouting in this model of quiescence. MitoC treatment could not rescue this effect, however lowering ROS levels using the anti-oxidant NAC did rescue the phenotype. Similar results were obtained when inducing quiescence using 3PO treatment. Using a mosaic model mixing 50%WT and 50% NICD^{OE} cells excluded the NICD overexpressing cells from the tip and concomitant CPT1a KD in these cells aggravated the phenotype. Strikingly, mitoC treatment could not rescue this effect while anti-oxidant treatment did. Conversely we also assessed the effect of CPT1a^{OE}, which induced FAO. In regular angiogenic ECs, overexpression of CPT1a induced sprouting and even more, it aggravated the hypersprouting induced by Notch blockade using DAPT or NOTCH1^{KD}.

### 7. Selective inhibition of CPT1a can be used to treat ocular angiogenesis in an animal model for age-related macular degeneration

The protein extravasation and hemorrhage associated with choroidal neovascularization (CNV) are primary causes of severe vision loss in retinal diseases such as age-related macular degeneration (ARMD). In ARMD the normal barrier function of Bruch's membrane is compromised, and CNV can develop either under the retinal pigment epithelium (RPE) and photoreceptor outer segments. The choroidal neovascularization model serves as a reliable disease model for macular degeneration. Choroidal neovascularization (CNV) is induced in mice by laser burn. Laser burn (400 mW) is performed with Alcon Purepoint equipment. CNV is measured by investigators masked to treatment. Eyes are enucleated after retrobulbar perfusion with FITC-dextran (HMW) and flat mounted. The CNV area, total lesion area, and their ratio are analyzed using Zeiss Axio Imager Z1 microscope with macros (KS300 image analysis software) on FITC-perfused (200 µL; 25 mg/mL; 10 min) flat mounts. The intraocular administration of siRNAs directed against CPT1a or the use of chemical inhibitors against CPT1a, is carried out, prior or shortly after the induction of the laser burn, in the above described murine model for age-related macular degeneration. Intraocular delivery of small interfering RNAs specific for CPT1a to the eye of these mice is accomplished by delivery of a specific small interfering RNA for CPT1a into the eye via intraocular delivery. Representative examples of siRNA sequences directed against murine CPT1a are used. Alternatively the sequences are modified with phosphorothioate modifications throughout and 2'-O-(2-methoxy)ethyl substitutions on the sugars of the first and last 5 nucleotides to increase biological half-lives and binding affinity. Clinical analysis of the mice is carried out to confirm the effect on the development of pathological angiogenesis of knocking down the activity of CPT1a in the eye by either siRNAs directed against CPT1a or by use of a chemical inhibitor of CPT1a.

### 8. Etomoxir, an inhibitor of carnitine palmitoyltransferase 1, reduces pathological ocular angiogenesis

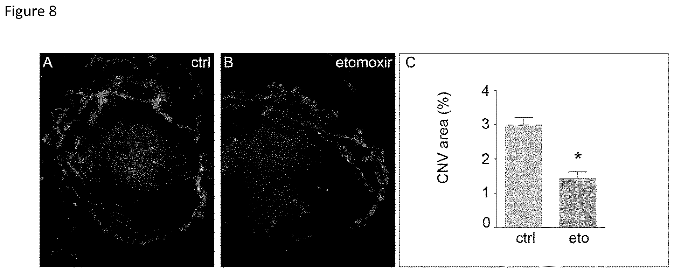

Choroidal neovascularization (CNV) was induced in male C57BL/6 mice by laser burn as previously described (Van de Veire S et al (2010) Cell 141(1):178-90). Using a Purepoint® Laser (Alcon, Fort Worth, United States), three spots were made on the retina in a star shaped way (0.4 Watt, 0.1 sec, 50 µM spot size). Mice were randomly allocated to the treatment groups and injected i.p. with vehicle or 35mg/kg etomoxir daily. After two weeks, the eyes were enucleated 1 minute after retrobulbar injection with Fluorescein isothiocyanate (FITC)-conjugated dextran (Mr 2,000,000) (Sigma), fixed in 4% PFA and choroids were dissected and flat-mounted for analysis of the neovascular lesion area. As shown in figure 8, etomoxir reduced the pathological neovascular area when compared to vehicle treated mice (n=6 mice for ctrl, n=7 mice for 35mg/kg; *p<0.05). Panel A and B show a representative image of a control (A) and etomoxir (B) treated CNV lesion, panel C shows the quantification of the CNV area. The data clearly show a reduction of less than 50% neovascularization area in the etomoxir treated mice.

## Claims

1. A compound inhibiting the activity of carnitine palmitoyltransferase 1A (CPT1 a) for use in the treatment of pathological ocular angiogenesis such as age-related macular degeneration, diabetic retinopathy, diabetic maculopathy, proliferative retinopathies, choroidal and other intraocular disorders with an excessive angiogenesis component.

2. A compound for use in the treatment of pathological ocular angiogenesis according to claim 1 which compound is selected from the list consisting of a siRNA directed against carnitine palmitoyltransferase 1A, dsRNA directed against carnitine palmitoyltransferase 1A, anti-sense directed against carnitine palmitoyltransferase 1A, a ribozyme directed against carnitine palmitoyltransferase 1A, a microRNA directed against carnitine palmitoyltransferase 1A and chemical inhibitors of palmitoyltransferase 1A.

3. A pharmaceutical ophthalmic composition comprising a compound and a pharmaceutically acceptable carrier for use according to claims 1 or 2.

4. A pharmaceutical ophthalmic composition for use according to claim 3 for the treatment of pathological angiogenesis wherein said pathological angiogenesis is associated with age-related macular degeneration, diabetic retinopathy, diabetic maculopathy, proliferative retinopathies, choroidal and other intraocular disorders with an excessive angiogenesis component.

## Patentansprüche

1. Verbindung, die die Aktivität von Carnitinpalmitoyltransferase 1A (CPT1a) hemmt, zur Verwendung bei der Behandlung von pathologischer okulärer Angiogenese, wie altersbedingter Makuladegeneration, diabetischer Retinopathie, diabetischer Makulopathie, proliferativen Retinopathien, Chorion- und anderen intraokulären Erkrankungen mit einer übermäßigen Angiogenesekomponente.

2. Verbindung zur Verwendung bei der Behandlung von pathologischer okulärer Angiogenese nach Anspruch 1, wobei diese Verbindung aus der Liste bestehend aus einer siRNA, die gegen Carnitinpalmitoyltransferase 1A gerichtet ist, dsRNA, die gegen Carnitinpalmitoyltransferase 1A gerichtet ist, Antisense, die gegen Carnitinpalmitoyltransferase 1A gerichtet ist, einem Ribozym, das gegen Carnitinpalmitoyltransferase 1A gerichtet ist, einer microRNA, die gegen Carnitinpalmitoyltransferase 1A gerichtet ist, und chemischen Hemmern von Palmitoyltransferase 1A ausgewählt ist.

3. Pharmazeutische ophthalmische Zusammensetzung, die eine Verbindung und einen pharmazeutisch akzeptablen Trägerstoff umfasst, zur Verwendung nach Anspruch 1 oder 2.

4. Pharmazeutische ophthalmische Zusammensetzung zur Verwendung nach Anspruch 3 zur Behandlung von pathologischer Angiogenese, wobei die pathologische Angiogenese mit altersbedingter Makuladegeneration, diabetischer Retinopathie, diabetischer Makulopathie, proliferativen Retinopathien, Chorion- und anderen intraokulären Erkrankungen mit einer übermäßigen Angiogenesekomponente assoziiert ist.

## Revendications

1. Composé inhibant l'activité de carnitine palmitoyltransférase 1A (CPT1a) destiné à être utilisé dans le traitement de l'angiogenèse oculaire pathologique telle que la dégénérescence maculaire liée à l'âge, la rétinopathie diabétique, la maculopathie diabétique, des rétinopathies prolifératives, des troubles choroïdiens et autres troubles intraoculaires avec une composante d'angiogenèse excessive.

2. Composé destiné à être utilisé dans le traitement de l'angiogenèse oculaire pathologique selon la revendication 1, lequel composé est sélectionné parmi la liste constituée d'un ARNsi dirigé contre la carnitine palmitoyltransférase 1A, ARN à double brin dirigé contre la carnitine palmitoyltransférase 1A, anti-sens dirigé contre la carnitine palmitoyltransférase 1A, d'un ribozyme dirigé contre la carnitine palmitoyltransférase 1A, d'un microARN dirigé contre la carnitine palmitoyltransférase 1A et d'inhibiteurs chimiques de la palmitoyltransférase 1A.

3. Composition ophtalmique pharmaceutique comprenant un composé et un vecteur pharmaceutiquement acceptable destinée à être utilisée selon la revendication 1 ou 2.

4. Composition ophtalmique pharmaceutique destinée à être utilisée selon la revendication 3 pour le traitement de l'angiogenèse pathologique, dans laquelle ladite angiogenèse pathologique est associée à la dégénérescence maculaire liée à l'âge, la rétinopathie diabétique, la maculopathie diabétique, des rétinopathies prolifératives, des troubles choroïdiens et autres troubles intraoculaires avec une composante d'angiogenèse excessive.
